Europäisches Patentamt

European Patent Office

Office européen des brevets

(19)

(11) Veröffentlichungsnummer: **0 052 307**
**A1**

# (12) EUROPÄISCHE PATENTANMELDUNG

(21) Anmeldenummer: **81109497.8**

(22) Anmeldetag: **04.11.81**

(51) Int. Cl.³: **C 07 C 143/60**

(30) Priorität: **15.11.80 DE 3043126**

(43) Veröffentlichungstag der Anmeldung:
**26.05.82 Patentblatt 82/21**

(84) Benannte Vertragsstaaten:
**CH DE FR GB IT LI**

(71) Anmelder: **BAYER AG**
**Zentralbereich Patente, Marken und Lizenzen**
**D-5090 Leverkusen 1, Bayerwerk(DE)**

(72) Erfinder: **Blank, Heinz Ulrich, Dr.**
**Am Geusfelde 35**
**D-5068 OdenthaL(DE)**

(72) Erfinder: **Behre, Horst, Dr.**
**Zur alten Linde 12**
**D-5068 Odenthal-Eikamp(DE)**

(72) Erfinder: **Linden, Hans Werner, Dr.**
**Heymannstrasse 38**
**D-5090 Leverkusen(DE)**

(72) Erfinder: **Mentzel, Werner, Dr.**
**Morgengraben 5**
**D-5000 Köln 80(DE)**

(54) **Verfahren zur Herstellung von 1-Naphthylamin-4.6-disulfonsäure und 1-Naphthylamin-2.4.6-trisulfonsäure.**

(57) Neues Verfahren zur Herstellung von 1-Napthylamin-4.6-disulfonsäure und 1-Naphthylamin-2.4.6-trisulfonsäure gemäß dem man 1-Naphthylamin-6-sulfonsäure in Gegenwart von Alkali-, Erdalkali- und/oder Ammoniumionen abgebenden Substanzen mit Oleum sulfoniert und anschließend das Sulfonierungsgemisch entweder in üblicher Weise aufarbeitet (Herstellung von 1-Naphthylamin-4.6-disulfonsäure) oder aber in einer zweiten Sulfonierungsstufe mit frischem Oleum umsetzt und dann das so erhaltene Sulfonierungsgemisch in üblicher Weise aufarbeitet (Herstellung von 1-Naphthlyamin-2.4.6-trisulfonsäure).

EP 0 052 307 A1

Croydon Printing Company Ltd.

BAYER AKTIENGESELLSCHAFT     5090 Leverkusen, Bayerwerk

Zentralbereich              B/bc/c
Patente, Marken und Lizenzen


Verfahren zur Herstellung von 1-Naphthylamin-4.6-
disulfonsäure und 1-Naphthylamin-2.4.6-trisulfonsäure

Die Erfindung betrifft ein neues Verfahren zur Herstellung von 1-Naphthylamin-4.6-disulfonsäure und 1-
Naphthylamin-2.4.6-trisulfonsäure.

1-Naphthylamin-4.6-disulfonsäure und 1-Naphthylamin-2.4.6-
trisulfonsäure sind wichtige Zwischenprodukte für die
Herstellung von Farbstoffen.

Es sind bereits verschiedene Verfahren zur Herstellung
von 1-Naphthylamin-4.6-disulfonsäure bekannt. So ist
z.B. in der FR-PS 1 490 508 ein Verfahren zur Herstellung
von 1-Naphthylamin-4.6-disulfonsäure beschrieben, gemäß
dem man 1-Naphthylamin-6-sulfonsäure mit Mangandioxid
und Natriumhydrogensulfit sulfoniert. Dieses Verfahren
hat jedoch den Nachteil, daß teures Mangandioxid verwendet wird, eine schlechte Raum/Zeit-Ausbeute erzielt
wird und große Mengen salzhaltiger Mutterlauge anfallen.

Le A 20 629-Ausland

- 2 -

Es kommt daher für eine Herstellung von 1-Naphthylamin-4.6-disulfonsäure in technischem Maßstab nicht in Betracht.

Von technischem Interesse sind die Verfahren zur Herstellung von 1-Naphthylamin-4.6-disulfonsäure, bei denen die Sulfonierung von 1-Naphthylamin-6-sulfonsäure mit Oleum vorgenommen wird. Solche Verfahren sind z.B. in Beilstein H, XIV, Seite 790; Ullmann, Enzyklopädie der Technischen Chemie, Band 12, Seite 628 (1960); DE-P-Anmeldung C 4021 (1891) referiert in Friedländer 33, Seite 432; und der GB-PS 15223 beschrieben.

Gemäß den Angaben in Beilstein soll sich 1-Naphthylamin-4.6-disulfonsäure durch Erhitzen von 1-Naphthylamin mit überschüssigem 25 %igem Oleum auf 120°C oder durch mehrtägige Einwirkung von 25 %igem Oleum auf 1-Naphthylamin-4-sulfonsäure bei Temperaturen unter 30°C bilden. Gemäß Ullmann wird 1-Naphthylamin-4.6-disulfonsäure durch Eintragen von 1-Naphthylamin-4-sulfonsäure in 25 %iges Oleum und 25-stündiges Nachrühren bei 30°C erhalten. Gemäß der in Friedländer referierten deutschen Patentanmeldung 4021 wird 1-Naphthylamin-4.6-disulfonsäure durch Behandeln von 1-Naphthylamin-6-sulfonsäure mit etwa 10 %igem Oleum bei 100 bis 150°C erhalten. Diese Verfahren haben jedoch den Nachteil, daß bei ihnen schwer trennbare Gemische aus beispielsweise 1-Naphthylamin-4.6- und -4.7-disulfonsäure entstehen.

Le A 20 629

Gemäß dem in der GB-PS 15223 beschriebenen Verfahren wird 1-Naphthylamin-6-sulfonsäure durch Eintragen in 25 %iges Oleum und nachfolgendes 3- bis 4-stündiges Wärmen der Sulfonierungsmischung auf 50 bis 60°C sulfoniert. 1-Naphthylamin-4.6-disulfonsäure wird jedoch nicht isoliert; das Sulfonierungsgemisch wird vielmehr unmittelbar weiter zu 1-Naphthylamin-2.4.6-trisulfonsäure sulfoniert. Die Nacharbeitung dieses Sulfonierungsverfahrens ergab, daß bei diesem beträchtliche Mengen an unerwünschter 1-Naphthylamin-3.6-disulfonsäure und 1-Naphthylamin-2.4.6-trisulfonsäure entstehen. Wegen der schlechten Ausbeuten an der gewünschten 1-Naphthylamin-4.6-disulfonsäure und der Schwierigkeiten bei der Isomerentrennung kommt daher diesem Verfahren für eine technische Produktion keine Bedeutung zu.

Es wurde nun gefunden, daß sich die Ausbeute an 1-Naphthylamin-4.6-disulfonsäure erheblich erhöhen läßt, wenn man die Sulfonierung der 1-Naphthylamin-6-sulfonsäure in Gegenwart von Alkali-, Erdalkali- und/oder Ammoniumionen abgebenden Substanzen durchführt.

Die Erfindung betrifft daher ein Verfahren zur Herstellung von 1-Naphthylamin-4.6-disulfonsäure durch Sulfonieren von 1-Naphthylamin-6-sulfonsäure mit Oleum, Aufarbeiten des Sulfonierungsgemisches durch Eintragen in Wasser und Isolieren der 1-Naphthylamin-4.6-disulfonsäure, das dadurch gekennzeichnet ist, daß man die Sulfonierung in Gegenwart von Alkali-, Erdalkali- und/oder Ammoniumionen abgebenden Substanzen durchführt.

Le A 20 629

Vorteilhaft wendet man die Alkali-, Erdalkali- und/oder Ammoniumionen abgebenden Substanzen in einer solchen Menge an, daß auf 1 Mol 1-Naphthylamin-6-sulfonsäure 0,5 bis 4 Val (= Grammäquivalent), vorzugsweise 0,5 bis 2 Val, Alkali-, Erdalkali- und/oder Ammoniumionen entfallen.

Als Alkali-, Erdalkali- und/oder Ammoniumionen abgebende Substanzen seien vor allem genannt: Saure und neutrale Natrium-, Kalium-, Magnesium- und Ammonium-Salze anorganischer Säuren, wie Salzsäure, Schwefelsäure, Phosphorsäure und organischer Säuren, z.B. Carbonsäuren wie der Essigsäure, oder Sulfonsäuren, wie der Toluolsulfonsäure. Besonders bewährt haben sich Alkali-, Erdalkali- und Ammoniumsulfate und -hydrogensulfate wie Natrium-, Kalium-, Ammonium- und Magnesiumsulfat oder Natrium-, Kalium-, Ammonium- und Magnesiumhydrogensulfat; ferner die Alkali-, Erdalkali- und Ammoniumsalze der 1-Naphthylamin-6-sulfonsäure.

Die vorgenannten Salze können sowohl in wasserfreier als auch in Hydratwasser enthaltender Form eingesetzt werden. Werden Hydratwasser enthaltende Salze eingesetzt, so ist über die zur Sulfonierung erforderliche Menge an Schwefeltrioxid hinaus soviel Schwefeltrioxid (in Form von Oleum) zuzusetzen, wie zum Binden des Hydratwassers als Schwefelsäure erforderlich ist.

Die Ammoniumionen können sich vom Ammoniak, aber auch von Aminen, wie Di- oder Triethylamin oder Cyclohexyl-

Le A 20 629

amin, ableiten.

Das zur Sulfonierung zu verwendende Oleum kann 20 bis 100 Gew.-% Schwefeltrioxid enthalten. Vorzugsweise wird ein Oleum verwendet, das 50 bis 80 Gew.-% freies Schwefeltrioxid enthält.

Der erfindungsgemäße Zusatz der Alkali-, Erdalkali- und/oder Ammoniumionen abgebenden Verbindungen bewirkt eine Verlangsammung der Sulfonierungsreaktion. Deshalb erfordern die erfindungsgemäß erhaltenen Sulfonierungsgemische bis zum vollständigen Umsatz der 1-Naphthylamin-6-sulfonsäure etwas längere Nachrührzeiten als die Sulfonierungsgemische, die in Abwesenheit besagte Ionen abgebender Verbindungen erhalten werden. Im allgemeinen hat es sich bewährt, wenn man das Sulfonierungsgemisch nach erfolgter Oleumzugabe bei Temperaturen von 20 bis 50°C, vorzugsweise 30 bis 40°C, 3 bis 30 Stunden, vorzugsweise 16 bis 24 Stunden unter Rühren ausreagieren läßt. Das Fortschreiten der Sulfonierungsreaktion wird analytisch, z.B. durch Hochdruckflüssigkeit-Chromatographie, verfolgt. Das Nachrühren wird beendet, wenn der Gehalt der 1-Naphthylamin-6-sulfonsäure im Reaktionsgemisch unter die Nachweisgrenze gesunken ist.

Besonders bewährt hat sich der erfindungsgemäße Zusatz der Alkali-, Erdalkali- und/oder Ammoniumionen abgebenden Substanzen bei der Sulfonierung, bei der man das Oleum bei einer Temperatur von 10 bis 70°C, vorzugsweise 20 bis 40°C, entweder gleichzeitig mit der 1-
Naphthylamin-6-sulfonsäure zu vorgelegter Schwefelsäure gibt oder, vorzugsweise, zu in Schwefelsäure
gelöster bzw. suspendierter 1-Naphthylamin-6-sulfonsäure gibt und ein solches Molverhältnis Schwefeltrioxid:1-Naphthylamin-6-sulfonsäure anwendet, daß
1,2 bis 3 Mol Schwefeltrioxid auf 1 Mol 1-Naphthylamin-6-sulfonsäure entfallen.

Zum Lösen, bzw. Suspendieren der 1-Naphthylamin-6-
sulfonsäure wird vorzugsweise 80 bis 100 %ige, insbesondere 96 bis 100 %ige Schwefelsäure verwendet.
Die Menge an zum Lösen, bzw. Suspendieren verwendeter
Schwefelsäure wird vorteilhaft so bemessen, daß auf
1 Mol 1-Naphthylamin-6-sulfonsäure 6 bis 8 Mol Schwefelsäure entfallen.

Wird zum Lösen, bzw. Suspendieren, wasserhaltige Schwefelsäure verwendet, oder keine trockene, sondern feuchte
1-Naphthylamin-6-sulfonsäure eingesetzt, so ist über die
Menge von 1,2 bis 3 Mol Schwefeltrioxid je Mol 1-Naphthylamin-6-sulfonsäure hinaus soviel Schwefeltrioxid
(in Form von Oleum) zuzusetzen, wie zum Binden des in
den Reagentien enthaltenden Wassers als Schwefelsäure
erforderlich ist.

Nach beendeter Sulfonierung wird die 1-Naphthylamin-4.6-
disulfonsäure aus dem Sulfonierungsgemisch in an sich
bekannter Weise durch Eintragen des Gemisches in Wasser

<u>Le A 20 629</u>

und Abfiltrieren der auskristallisierten Säure, bzw. nach Aussalzen, z.B. mit Kochsalz, dessen saure Natriumsalze in an sich bekannter Weise isoliert.

Durch die erfindungsgemäße Mitverwendung der Alkali-, Erdalkali- und/oder Ammoniumionen abgebenden Substanzen bei der Sulfonierung wird eine wesentliche Erhöhung der Ausbeute an 1-Naphthylamin-4.6-disulfonsäure und eine Abnahme der Menge an 1-Naphthylamin-3.6-disulfonsäure im Sulfonierungsgemisch erhalten.

Für den Fall der Weiterverarbeitung der 1-Naphthylamin-4.6-disulfonsäure zu 1-Naphthylamin-2.4.6-trisulfonsäure ist eine Isolierung der 1-Naphthylamin-4.6-disulfonsäure nicht erforderlich. Vielmehr wird das fertige Sulfonierungsgemisch in einer zweiten Sulfonierungsstufe bei Temperaturen von 70 bis 120°C, vorzugsweise 80 bis 100°C, mit einer solchen Menge Oleum versetzt, daß auf 1 Mol ursprünglich eingesetzte 1-Naphthylamin-6-sulfonsäure 1 bis 2,5 Mol, vorzugsweise 1,5 bis 2 Mol, Schwefeltrioxid entfallen. Nach beendeter Oleumzugabe läßt man das Sulfonierungsgemisch 2 bis 8 Stunden bei 70 bis 120°C, vorzugsweise 90 bis 110°C, ausreagieren. Man isoliert die entstandene 1-Naphthylamin-2.4.6-trisulfonsäure in an sich bekannter Weise durch Eintragen des Sulfonierungsgemisches in Wasser. Die Sulfonsäure wird als freie Säure, bzw. durch Aussalzen, z.B. mit Kochsalz, als saures Dinatriumsalz erhalten.

Infolge ihres wesentlich höheren Gehaltes an 1-Naphthylamin-4.6-disulfonsäure und geringem Gehalt an 1-Naphthylamin-3.6-disulfonsäure liefern die bei der erfindungsgemäßen Herstellung von 1-Naphthylamin-4.6-disulfonsäure anfallenden Sulfonierungsgemische 1-Naphthylamin-2.4.6-trisulfonsäure in höheren Ausbeuten und höherer Reinheit als die gemäß Stand der Technik erhältlichen, 1-Naphthylamin-4.6-disulfonsäure enthaltenden Sulfonierungsgemische.

Die Erfindung betrifft daher fernerhin ein Verfahren zur Herstellung von 1-Naphthylamin-2.4.6-trisulfonsäure.

Das Verfahren ist dadurch gekennzeichnet, daß man das bei der Herstellung von 1-Naphthylamin-4.6-disulfonsäure anfallende Sulfonierungsgemisch in einer zweiten Sulfonierungsstufe bei Temperaturen von 70 bis 120°C, vorzugsweise 90 bis 110°C, mit Oleum umsetzt, wobei man die Oleummenge so bemißt, daß 1 bis 2,5 Mol, vorzugsweise 1,5 bis 2 Mol Schwefeltrioxid auf 1 Mol ursprünglich eingesetzte 1-Naphthylamin-6-sulfonsäure entfallen.

Das zu verwendende Oleum kann 20 bis 100 Gew.-% Schwefeltrioxid enthalten. Vorzugsweise wird ein Oleum verwendet, das 50 bis 80 Gew.-% freies Schwefeltrioxid enthält.

Le A 20 629

Beispiel 1

In einem mit Rührer, Tropftrichter, Innenthermometer und Trockenrohr versehenen Reaktionsgefäß werden 760 g 100 %ige Schwefelsäure und 66 g (1 Val) wasserfreies Ammoniumsulfat vorgelegt. In diese Mischung werden unter Rühren innerhalb von 30 Minuten 227,8 g (1 Mol) 1-Naphthylamin-6-sulfonsäure (98 %ig) bei höchstens 40°C eingetragen. Dann werden in die gut rührbare Suspension innerhalb von 30 Minuten bei 30°C 307,5 g Oleum (65 %ig) (= 2,5 Mol $SO_3$) getropft. Die Sulfonierungsmischung wird 24 Stunden bei 30°C nachgerührt.

Dann wird das Sulfonierungsgemisch unter Rühren mit einer solchen Geschwindigkeit in 1520 g Wasser eingetragen, daß die Temperatur der entstehenden wäßrigen Mischung auf 90 bis 95°C steigt. Nach wenigen Minuten fällt die 1-Naphthylamin-4.6-disulfonsäure aus. Man läßt unter Rühren auf 20°C abkühlen (Abkühlzeit: 5 bis 6 Stunden) und filtriert. Der Filterkuchen wird gut abgepreßt (Auswaage: 367 g).

Die Hochdruck-Flüssigkeitschromatographie des abfiltrierten Produktes ergab folgende Zusammensetzung:

| | |
|---|---|
| 1-Naphthylamin-4.6-disulfonsäure | 74,4 Gew.-% |
| 1-Naphthylamin-2.4.6-trisulfonsäure | Spur |
| etwa 0,2 Gew.-% Ammoniumsulfat | |
| Rest bis 100 % | Wasser/Schwefelsäure |

Le A 20 629

Die Ausbeute an reiner 1-Naphthylamin-4.6-disulfonsäure beträgt 273 g (= 90 % der Theorie, bezogen auf eingesetzte 1-Naphthylamin-6-sulfonsäure).

Wird die Sulfonierung ohne den Zusatz an Ammoniumsulfat durchgeführt, so weist das abfiltrierte Produkt (Auswaage: 321 g) gemäß Hochdruck-Flüssigkeitschromatographie folgende Zusammensetzung auf:

| | |
|---|---|
| 1-Naphthylamin-4.6-disulfonsäure: | 76,1 Gew.-% |
| 1-Naphthylamin-3.6-disulfonsäure: | 0,1 Gew.-% |
| 1-Naphthylamin-2.4.6-trisulfonsäure: | Spur |
| Rest bis 100 %: | Wasser/Schwefelsäure |

und die Ausbeute an reiner 1-Naphthylamin-4.6-disulfonsäure beträgt nur 244,3 g (= 80,5 % der Theorie, bezogen auf eingesetzte 1-Naphthylamin-6-sulfonsäure).

Beispiele 2 bis 11

Es wird wie in Beispiel 1 beschrieben gearbeitet, nur werden andere Salze und Salzmengen, andere Molverhältnisse Schwefeltrioxid:1-Naphthylamin-6-sulfonsäure, andere Reaktionstemperaturen und Nachrührzeiten angewendet.

In der nachstehenden Tabelle sind die bei diesen Herstellungsverfahren erhaltenen Ergebnisse zusammengestellt. Die Zusammensetzung der isolierten 1-Naphthylamin-4.6-disulfonsäuren wurde mittels Hochdruck-Flüssigkeitschromatographie bestimmt.

Le A 20 629

Tabelle

| Beispiel-Nr. | Sulfonierungsbedingungen | | | | | Aus-waage $[g]$ | Isolierter Feststoff Gehalt an $[Gew.-\%]$ [1,2] | | | Ausbeute an 1-NH$_2$-4.6-disulfonsäure $[\%$ d. Theorie$]$ |
|---|---|---|---|---|---|---|---|---|---|---|
| | Molver-hältnis SO$_3$ : 1-NH$_2$-6-sulfonsäure | Salzzusatz | | Reak-tions-tempe-ratur $[°C]$ | Nach-rühr-zeit $[h]$ | | 1-NH$_2$-4.6-di-sulfonsäure | 1-NH$_2$-3.6-disulfon-säure | 1-NH$_2$-2.4.6-trisulfonsäure | |
| | | $[Val]$ | Salz | | | | | | | |
| 2 | 2,5:1 | 0,5 | (NH$_4$)$_2$SO$_4$ | 30 | 20 | 312,0 | 82,7 | – | Spur | 85,1 |
| 3 | 2,5:1 | 1,0 | Na$_2$SO$_4$ | 30 | 24 | 344,0 | 78,8 | – | 0,1 | 89,4 |
| 4 | 2,5:1 | 1,0 | K$_2$SO$_4$ | 30 | 24 | 350,5 | 76,6 | – | 0,2 | 88,5 |
| 5 | 2,0:1 | 1,0 | (NH$_4$)$_2$SO$_4$ | 40 | 24 | 338,4 | 80,3 | – | 0,4 | 89,6 |
| 6 | 2,0:1 | 1,0 | Na$_2$SO$_4$ | 40 | 24 | 340,0 | 79,8 | – | 1,2 | 89,5 |
| 7 | 2,0:1 | 1,5 | (NH$_4$)$_2$SO$_4$ | 40 | 28 | 342,0 | 78,3 | 0,2 | 2,4 | 89,0 |
| 8 | 2,0:1 | – | – | 30 | 16 | 321,0 | 76,1 | 0,1 | Spur | 80,6 |
| 9 | 2,0:1 | – | – | 40 | 8 | 308,0 | 76,7 | 0,6 | 0,2 | 77,9 |
| 10 | 2,0:1 | 1,0 | K$_2$SO$_4$ | 40 | 24 | 344,5 | 78,8 | 0,2 | 0,3 | 89,5 |
| 11 | 2,5:1 | – | – | 30 | 9 | 351,0 | 68,5 | 2,8 | Spur | 79,2 |

1) Bestimmt mittels Hochdruck-Flüssigkeitschromatographie; bei den an 100 % fehlenden Gew.-% handelt es sich um H$_2$O, H$_2$SO$_4$ und geringe Mengen Salz

2) Der Gehalt an 1-Naphthylamin-6-sulfonsäure lag unterhalb der Nachweisgrenze

- 12 -

Beispiel 12

In der in Beispiel 1 beschriebenen Sulfonierungsapparatur werden 760 g Monohydrat und 71 g (1 Val) wasserfreies Natriumsulfat vorgelegt. In diese Mischung werden 227,8 g (1 Mol) 1-Naphthylamin-6-sulfonsäure (98 %ig) bei höchstens 40°C eingetragen. Dann werden in die gut rührbare Suspension innerhalb von 30 Minuten bei ca. 30°C 246 g 65 %iges Oleum (= 2 Mol $SO_3$) getropft. Die Sulfonierungsmischung wird anschließend 22 Stunden bei 30°C gerührt.

Dann werden nochmals ohne zu Kühlen 246 g 65 %iges Oleum (= 2 Mol $SO_3$) zugegeben. Das Sulfonierungsgemisch wird unter Rühren 8 Stunden auf 100 bis 110°C erwärmt, dann auf 20 bis 30°C abgekühlt und mit einer solchen Geschwindigkeit in 2600 g Wasser eingetragen, daß die Temperatur der entstehenden wäßrigen Mischung auf 90 bis 100°C ansteigt. Man versetzt die wäßrige Mischung mit 250 g Kochsalz und kühlt im Verlauf von 5 bis 6 Stunden auf 20°C, saugt den Niederschlag ab und wäscht ihn mit gesättigter Kochsalzlösung, bis diese annähernd farblos abläuft. Der Feststoff wird im Vakuum-Trockenschrank bei 90°C getrocknet.
(Auswaage: 616,9 g).

Das getrocknete Produkt weist gemäß Hochdruck-Flüssigkeitschromatographie folgende Zusammensetzung auf:

Le A 20 629

1-Naphthylamin-2.4.6-trisulfonsäure: 57,3 Gew.-%
1-Naphthylamin-3.6-disulfonsäure:    0,7 Gew.-%
1-Naphthylamin-4.6-disulfonsäure:    0,1 Gew.-%
Rest bis 100 %:                  $NaCl, H_2O$

Die Ausbeute an reinem 1-Naphthylamin-2.4.6-trisulfonsäure-dinatriumsalz beträgt 353,5 g (= 92,3 % der Theorie, bezogen auf eingesetzte 1-Naphthylamin-6-sulfon-säure).

Le A 20 629

0052307

Patentansprüche

1) Verfahren zur Herstellung von 1-Naphthylamin-4.6-disulfonsäure durch Sulfonieren von 1-Naphthylamin-6-sulfonsäure mit Oleum, Aufarbeiten des Sulfonierungsgemisches durch Eintragen in Wasser und Isolieren der 1-Naphthylamin-4.6-disulfonsäure, dadurch gekennzeichnet, daß man die Sulfonierung in Gegenwart von Alkali-, Erdalkali- und/oder Ammoniumionen abgebenden Substanzen durchführt.

2) Verfahren gemäß Anspruch 1, dadurch gekennzeichnet, daß man die Alkali-, Erdalkali- und/oder Ammoniumionen abgebenden Substanzen in einer solchen Menge anwendet, daß auf 1 Mol 1-Naphthylamin-6-sulfonsäure 0,5 bis 4 Val Alkali-, Erdalkali- und/oder Ammoniumionen entfallen.

3) Verfahren gemäß Ansprüchen 1 und 2, dadurch gekennzeichnet, daß man die Alkali-, Erdalkali- und/oder Ammoniumionen abgebenden Substanzen in einer solchen Menge anwendet, daß auf 1 Mol 1-Naphthylamin-6-sulfonsäure 0,5 bis 2 Val Alkali-, Erdalkali- und/oder Ammoniumionen entfallen.

4) Verfahren gemäß Ansprüchen 1 bis 3, dadurch gekennzeichnet, daß man als Alkali-, Erdalkali- und/oder Ammoniumionen abgebende Substanzen saure und neutrale Natrium-, Kalium-, Magnesium-, und/oder Ammoniumsalze anorganischer oder organischer Säuren verwendet.

Le A 20 629

- 15 -

5) Verfahren gemäß Ansprüchen 1 bis 4, dadurch gekennzeichnet, daß als Alkali-, Erdalkali- und/oder Ammoniumionen abgebende Substanzen Natrium-, Kalium-, Ammonium- und/oder Magnesiumsulfat bzw. -hydrogensulfat bzw. 1-Naphthylamin-6-sulfonat eingesetzt werden.

6) Verfahren gemäß Ansprüchen 1 bis 5, dadurch gekennzeichnet, daß man die Sulfonierung in der Weise vornimmt, daß man das Oleum bei einer Temperatur von 10 bis 70°C entweder gleichzeitig mit der 1-Naphthylamin-6-sulfonsäure zu vorgelegter Schwefelsäure oder zu in Schwefelsäure gelöster bzw. suspendierter 1-Naphthylamin-6-sulfonsäure gibt und ein solches Molverhältnis Schwefeltrioxid zu 1-Naphthylamin-6-sulfonsäure anwendet, das 1,2 bis 3 Mol Schwefeltrioxid auf 1 Mol 1-Naphthylamin-6-sulfonsäure entfallen.

7) Verfahren zur Herstellung von 1-Naphthylamin-2.4.6-trisulfonsäure, dadurch gekennzeichnet, daß man das bei der Herstellung von 1-Naphthylamin-4.6-disulfonsäure gemäß Anspruch 1 bis 6 anfallende Sulfonierungsgemisch in einer zweiten Sulfonierungsstufe bei Temperaturen von 70 bis 120°C mit Oleum umsetzt, wobei man die Oleum-Menge so bemißt, daß 1 bis 2,5 Mol Schwefeltrioxid auf 1 Mol 1-Naphthylamin-6-sulfonsäure entfallen.

Le A 20 629

**Europäisches Patentamt**

**EUROPÄISCHER RECHERCHENBERICHT**

Nummer der Anmeldung

EP 81 10 9497.8

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| D,A | <u>FR - A - 1 490 508</u>  (KUHLMANN) <br> * Beispiel 7 * <br> -- | 1-7 |
| A | Chemical Abstracts Band 63, Nr. 7, <br> 27. September 1965 <br> Columbus, Ohio, USA <br> J. HORYNA et al. "Isolating 1-naphthyl-amine-4,6-disulfonic and 1-naphthylamine-2,4,6-trisulfonic acid" <br> Spalte 8288b-d <br> & CS - A - 112946 <br> -- | |
| A | Chemical Abstracts Band 71, Nr. 9 <br> 1. September 1969 <br> Columbus, Ohio, USA <br> J. HORYNA et al. "1-Aminonaphthalene-sulfonic acids" <br> Seite 258, linke Spalte, Abstract <br> Nr. 38659a <br> & CS - A - 129210 <br> -- | |
| D,A | Beilsteins Handbuch der organischen Chemie <br> 4. Auflage, Band XIV <br> 1931, VERLAG VON JULIUS SPRINGER, Berlin <br> Seite 790 <br> ----- | |

**KLASSIFIKATION DER ANMELDUNG (Int. Cl.³)**

C 07 C 143/60

**RECHERCHIERTE SACHGEBIETE (Int. Cl.³)**

C 07 C 143/00

**KATEGORIE DER GENANNTEN DOKUMENTE**

X: von besonderer Bedeutung

A: technologischer Hintergrund

O: nichtschriftliche Offenbarung

P: Zwischenliteratur

T: der Erfindung zugrunde liegende Theorien oder Grundsätze

E: kollidierende Anmeldung

D: in der Anmeldung angeführtes Dokument

L: aus andern Gründen angeführtes Dokument

&: Mitglied der gleichen Patent-familie, übereinstimmendes Dokument

Der vorliegende Recherchenbericht wurde für alle Patentansprüche erstellt.

| Recherchenort | Abschlußdatum der Recherche | Prüfer |
|---|---|---|
| Berlin | 08-02-1982 | BREW |